# FASCICULE DE BREVET EUROPEEN

(11) **EP 1 230 252 B1**
(45) Date de publication et mention de la délivrance du brevet: **14.04.2004**
(21) Numéro de dépôt: 00979738.2
(22) Date de dépôt: 15.11.2000
(51) Int. Cl.: C07H 17/075, A61K 31/70

(54) **DERIVES DE BETA-D-5-THIOXYLOSE, PROCEDE DE PREPARATION ET UTILISATION EN THERAPEUTIQUE**
BETA-D-THIOXYLOS-DERIVATE, VERFAHREN ZUR HERSTELLUNG UND VERWENDUNG ALS ARZNEIMITTEL
BETA-D-5-THIOXYLOSE DERIVATIVES, PREPARATION METHOD AND THERAPEUTIC USE

(30) Priorité: 17.11.1999 FR 9914445
(43) Date de publication de la demande: 14.08.2002
(73) Titulaire: FOURNIER INDUSTRIE ET SANTE, 21300 Chenôve (FR)
(72) Inventeur: BARBEROUSSE, Véronique, F-21121 Hauteville-les-Dijon (FR); BOUBIA, Benaissa, F-21850 Saint-Apollinaire (FR); SAMRETH, Soth, F-21121 Daix (FR)
(74) Mandataire: Gillard, Marie-Louise
(86) Numéro de dépôt international: PCT/FR2000/003174
(87) Numéro de publication internationale: WO 2001/036437

(56) Documents cités:
- EP-A- 0 421 829

## Description

La présente invention concerne en tant que produits industriels nouveaux des dérivés de β-D-5-thioxylose, plus particulièrement les dérivés de 4-méthyl-2-oxo-2*H*-1-benzopyran-7-yl 5-thio-β-D-xylopyranoside de formule I ci-après. Elle concerne également le procédé de préparation et l'utilisation en thérapeutique de ces nouveaux produits.

### Art antérieur

On connaît de EP-A-0421829 et notamment du tableau I de ce document des composés benzopyranone β-D-thioxylosides de formule Io : 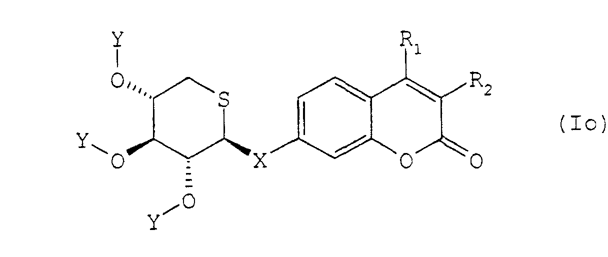 dans laquelle
X est O ou S,
Y est notamment H ou COCH₃,
R₁ est notamment alkyle en C₁-C₄, et
R₂ est en particulier H, alkyle en C₁-C₄ ou halogène,
qui sont présentés comme étant utiles en thérapeutique eu égard à leurs effets dans la prévention ou le traitement des troubles de la circulation veineuse, notamment les thromboses veineuses.

En particulier, le composé 4-méthyl-2-oxo-2*H*-1-benzopyran-7-yl 5-thio-β-D-xylopyranoside (formule Io où X = O, Y = H, R₁ = CH₃ et R₂ = H), administré par voie orale, présente une activité remarquable dès la dose de 6 mg/kg en ce sens qu'il réduit de plus de 80 % la formation d'un thrombus veineux. Cette activité, obtenue après administration par voie orale, est extrêmement intéressante dans le cas de l'utilisation la plus courante de tels produits et qui correspond généralement à un traitement préventif.

En effet, les produits couramment utilisés actuellement dans ce domaine en thérapeutique, par exemple les anticoagulants que sont les héparines normales et les héparines de bas poids moléculaire, ne sont pas actifs par voie orale et doivent être administrés par injection par voie intraveineuse ou sous-cutanée. Un tel mode d'administration, notamment lors d'un traitement chronique, est généralement peu apprécié par le patient qui préfère absorber par voie orale un comprimé ou une gélule. De ce point de vue, les composés selon EP-A-0421829 apportent un progrès certain pour le confort du patient, pour le risque et pour les coûts liés au mode d'administration. Il faut noter également que ces composés, contrairement aux produits déjà utilisés, n'augmentent pas le risque hémorragique.

Toutefois, les composés décrits dans EP-A-0421829 présentent une solubilité insuffisante pour permettre leur utilisation par voie injectable. De ce fait, leur utilisation n'est pas possible dans les cas où la voie injectable est la seule voie d'administration possible ou si, par commodité, il semble plutôt préférable d'administrer l'un de ces composés en association avec d'autres médicaments par perfusion.

### But de l'invention

Selon l'invention, on propose une nouvelle solution technique pour résoudre le problème précité de la solubilité. Cette nouvelle solution fait appel à de nouveaux dérivés de 4-méthyl-2-oxo-2*H-*1-benzopyran-7-yl 5-thio-β-D-xylopyranoside, qui présentent une meilleure solubilité dans les solvants usuels, notamment les solutés injectables, tout en conservant une activité par voie orale.

### Objet de l'invention

Selon l'invention on fournit, en tant que produit industriel nouveau, un composé caractérisé en ce qu'il répond à la formule I : 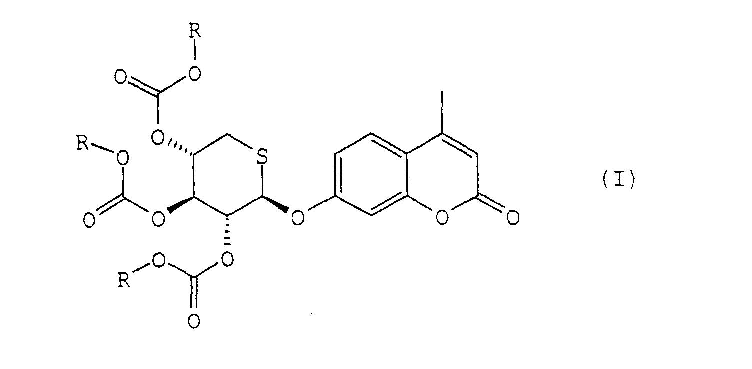 dans laquelle R représente un groupe alkyle en C₁-C₅ qui est linéaire, ramifié ou cyclisé, un groupe alcène en C₂-C₃ mono insaturé, un groupe hydroxyalkyle en C₂-C₃ ou un groupe alkoxyalkyle en C₃-C₆.

Selon un autre aspect de l'invention, on fournit un procédé de préparation d'un composé de formule I, ledit procédé étant caractérisé en ce qu'il comprend la réaction de carbonatation du 4-méthyl-2-oxo-2*H*-1-benzopyran-7-yl 5-thio-β-D-xylopyranoside de formule II : 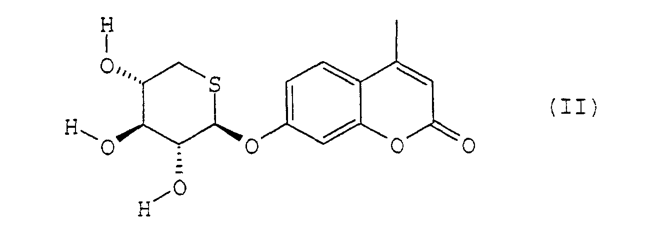 avec un réactif choisi parmi l'ensemble constitué par un halogénoformiate de formule III : 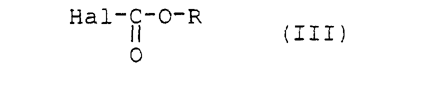 et un pyrocarbonate de formule IV : 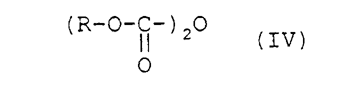 où R représente un groupe alkyle en C₁-C₅, un groupe alcène en C₂-C₃, un groupe alkoxyalkyle en C₃-C₆ ou un groupe hydroxyalkyle en C₂-C₃ dont la fonction alcool est protégée par un groupe protecteur tel que par exemple un groupe trialkylsilyle, et Hal est un atome d'halogène (notamment F, Cl ou Br, l'halogène préféré étant ici le chlore).

Ce procédé implique la protection de la fonctionnalité OH du groupe R = hydroxyalkyle en C₂-C₃ avant la carbonatation, puis sa déprotection après ladite carbonatation.

Selon la nature du groupe R, on procède comme suit. Quand R = R' = un groupe alkyle en C₁-C₅ qui est linéaire, ramifié ou cyclisé, un groupe alcène en C₂-C₃ mono insaturé, ou un groupe alkoxyalkyle en C₃-C₆, on met en oeuvre la réaction II + III ou II + IV.

Quand R = R" = groupe hydroxyalkyle en C₂-C₃, on met en oeuvre la réaction de carbonatation du 4-méthyl-2-oxo-2H-1-benzopyran-7-yl 5-thio-β-D-xylopyranoside de formule II avec un halogénoformiate de formule IIIbis : 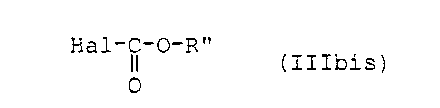 où R" est un groupe hydroxyalkyle en C₂-C₃ dans lequel la fonctionnalité OH est protégée, et Hal est un atome d'halogène comme défini ci-dessus, puis
l'on soumet le composé résultant à une réaction de déprotection du groupe hydroxy, par exemple si le groupe protecteur est un groupe trialkylsilyle, par action d'une solution d'acide fluorhydrique afin d'obtenir le composé de formule I dans laquelle R représente le groupe hydroxyalkyle en C₂-C₃.

Selon encore un autre aspect de l'invention, on fournit une composition pharmaceutique caractérisée en ce qu'elle comprend, en association avec un excipient physiologiquement acceptable, une quantité thérapeutiquement efficace d'au moins un composé de formule I, d'une part, et l'utilisation d'un composé de formule I, caractérisée en ce que l'on fait appel à un dit composé de formule I pour l'obtention d'un médicament antithrombotique destiné à une utilisation thérapeutique vis-à-vis des troubles de la circulation veineuse, d'autre part.

### Description détaillée de l'invention

Parmi les groupes alkyle en C₁-C₅, qui conviennent selon l'invention, on peut citer les groupes méthyle, éthyle, propyle, 1-méthyléthyle, cyclopropyle, butyle, 1-méthylpropyle, 2-méthylpropyle, 1,1-diméthyléthyle et pentyle.

Parmi les groupes alcène en C₂-C₄, qui conviennent selon l'invention, on peut citer les groupes vinyle et allyle. Un groupe alcène mono-insaturé désigne ici un groupe aliphatique comportant une seule double liaison C=C.

Parmi les groupes alkoxyalkyle en C₃-C₆, qui conviennent selon l'invention, on peut citer les groupes méthoxyéthyle, éthoxyéthyle, méthoxyéthoxyéthyle et éthoxyéthoxyéthyle.

Parmi les groupes hydroxyalkyle en C₂-C₃ qui conviennent selon l'invention, on peut citer les groupes 2-hydroxyéthyle, 2-hydroxypropyle ou 3-hydroxypropyle.

Parmi les composés de formule I, qui sont des tri-carbonates du 4-méthyl-2-oxo-2*H*-1-benzopyran-7-yl 5-thio-β-D-xylopyranoside, on préfère ceux dans lesquels R représente un groupe éthyle ou un groupe méthyle.

Les composés de formule I peuvent être préparés avantageusement selon
a) une réaction de carbonatation selon laquelle on fait réagir le 4-méthyl-2-oxo-2*H*-1-benzopyran-7-yl 5-thio-β-D-xylopyranoside de formule II : 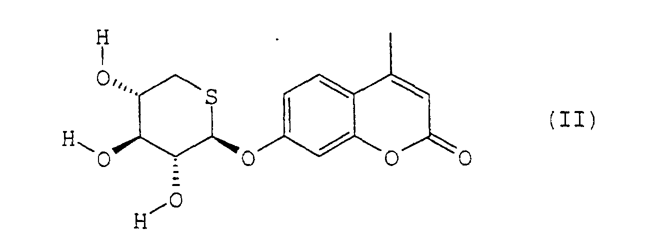 avec un chloroformiate de formule IIIa : 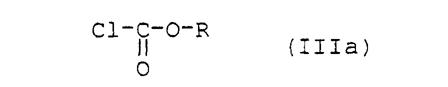 ou un pyrocarbonate de formule IV : 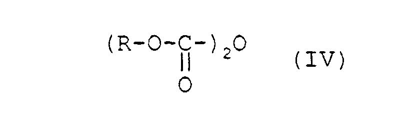 où R représente un groupe alkyle en C₁-C₅, linéaire, ramifié ou cyclisé, un groupe alcène en C₂-C₃ mono insaturé, un groupe alkoxyalkyle en C₃-C₆ ou un groupe hydroxyalkyle en C₂-C₃ dont le groupe hydroxy est protégé par un groupe protecteur, notamment le groupe t-butyldiméthylsilyle, la réaction étant conduite dans un solvant anhydre, tel que par exemple la diméthylformamide, en présence d'une base aprotique telle que par exemple la 4-(diméthylamino)pyridine, à une température comprise entre 10°C et 80°C et pendant 1 à 10 heures, pour obtenir le composé de formule I dans laquelle R conserve la même signification que dans le réactif IIIa ou IV, et
b) si nécessaire, dans le cas de la préparation d'un composé de formule I dans laquelle R est un groupe hydroxyalkyle, une réaction de déprotection du groupe hydroxy, notamment, si le groupe protecteur est un groupe t-butyldiméthylsilyle, par action de l'acide fluorhydrique, à température ambiante et dans un solvant tel que par exemple l'acétonitrile.

Quand R est un groupe hydroxyalkyle en C₂-C₃, on préfère que la réaction de carbonatation soit effectuée au moyen d'un chloroformiate. Le groupe protecteur de la fonctionnalité OH doit être plus facilement clivable que la fonction O-CO-O de façon à être enlevé lors de la déprotection sans affecter ladite fonction O-CO-O, le groupe protecteur préféré étant un groupe trialkylsilyle.

La composition thérapeutique peut être sous forme d'une solution ou d'une préparation permettant de réaliser une solution administrable par injection, soit sous-cutanée, soit intraveineuse, soit encore sous forme d'une perfusion. La composition thérapeutique peut également se présenter sous une forme administrable par voie orale, par exemple des gélules, des comprimés ou une solution buvable.

Les composés de formule I sont utiles en thérapeutique en raison de leur activité antithrombotique, et présentent un intérêt particulier pour le traitement ou la prévention des troubles de la circulation veineuse, notamment pour corriger certains paramètres hématologiques sensibles au niveau veineux.

Les exemples suivants, ainsi que les résultats d'essais pharmacologiques, nullement limitatifs, permettront de mieux comprendre l'intérêt de l'invention.

### Exemple 1

### 4-Méthyl-2-oxo-2H-1-benzopyran-7-yl 2,3,4-tri-O-méthoxycarbonyl-5-thio-β-D-xylopyranoside

On prépare une solution de 2 g (6,17.10⁻³ mole) de 4-méthyl-2-oxo-2*H*-1-benzopyran-7-yl 5-thio-β-D-xylopyranoside dans 20 ml de diméthylformamide à 70°C, puis on refroidit jusqu'à 25°C environ. On ajoute 10 mg (0,8.10⁻³ mole) de 4-diméthylaminopyridine, puis 3,85 ml (36.10⁻³ mole) de pyrocarbonate de diméthyle. Après 4 heures sous agitation à température ambiante (15-25°C), on ajoute à nouveau 3,85 ml de pyrocarbonate de diméthyle et on maintient le milieu réactionnel sous agitation à température ambiante pendant 6 heures. Le mélange est alors filtré, concentré sous pression réduite et le résidu est purifié par chromatographie sur gel de silice en éluant avec un mélange toluène-acétone (6/1 ; v/v). La fraction pure est cristallisée dans un mélange acétone-éther, filtrée et séchée. On obtient ainsi 1,92 g du produit attendu sous forme d'une poudre blanche (rendement = 63 %).
F = 168°C
[α]²¹_{D} = - 77° (c = 0,37 ; CHCl₃)

### Exemple 2

### 4-Méthyl-2-oxo-2H-1-benzopyran-7-yl 2,3,4-tri-O-éthoxycarbonyl-5-thio-β-D-xylopyranoside

En opérant de façon analogue à l'exemple 1, mais en remplaçant le pyrocarbonate de diméthyle par le pyrocarbonate de diéthyle, on obtient le produit attendu sous forme d'une poudre blanche (rendement = 78 %).
F = 176°C
[α]²²_{D} = - 72° (c = 0,465 ; CHCl₃)

### Exemple 3

### 4-Méthyl-2-oxo-2H-1-benzopyran-7-yl 2,3,4-tri-O-propoxycarbonyl-5-thio-β-D-xylopyranoside

On prépare une solution de 100 mg (0,8.10⁻³ mole) de 4-diméthylaminopyridine dans 12 ml de pyridine et on ajoute à 0°C, 2,1 ml (18,7.10⁻³ mole) de chloroformiate de propyle, puis 1 g (3,1.10⁻³ mole) de 4-méthyl-2-oxo-2*H*-1-benzopyran-7-yl 5-thio-β-D-xylopyranoside. Le mélange réactionnel est maintenu sous agitation pendant 1 heure, puis on ajoute à nouveau 1 ml (8,9.10⁻³ mole) de chloroformiate de propyle, et on agite encore pendant 1 heure. Le mélange est ensuite hydrolysé sur de l'eau glacée, et extrait par l'acétate d'éthyle. La phase organique est lavée avec une solution diluée d'acide chlorhydrique, puis à l'eau, séchée et enfin concentrée sous pression réduite. Le résidu est purifié par chromatographie sur gel de silice en éluant avec un mélange toluène/acétate d'éthyle (4/1 ; v/v). La fraction de produit pur est cristallisée dans le mélange acétate d'éthyle/éther diéthylique. On obtient ainsi 1,13 g du produit attendu, sous forme d'un solide fin et léger blanc (rendement = 63 %).
F = 134°C
[α]²³_{D} = - 57° (c = 0,37 ; CHCl₃)

### Exemple 4

### 4-Méthyl-2-oxo-2H-1-benzopyran-7-yl 2,3,4-tri-O-butoxycarbonyl-5-thio-β-D-xylopyranoside

En opérant de façon analogue à l'exemple 3, mais en remplaçant le chloroformiate de propyle par le chloroformiate de butyle, on obtient le produit attendu après cristallisation dans l'éther diéthylique, sous forme d'un solide cotonneux blanc (rendement = 85 %).
F = 120°C
[α]²³_{D} = - 51° (c = 0,45 ; CHCl₃)

### Exemple 5

### 4-Méthyl-2-oxo-2H-1-benzopyran-7-yl 2,3,4-tri-O-(2-méthylpropyloxycarbonyl)-5-thio-β-D-xylopyranoside

En opérant de façon analogue à l'exemple 3, mais en remplaçant le chloroformiate de propyle par le chloroformiate de 2-méthylpropyle, on obtient le produit attendu sous forme d'un solide fin blanc (rendement = 89 %).
F = 114°C
[α]²³_{D} = - 43° (c = 0,415 ; CHCl₃)

### Exemple 6

### 4-Méthyl-2-oxo-2H-1-benzopyran-7-yl 2,3,4-tri-O-(2-méthoxyéthoxycarbonyl)-5-thio-β-D-xytopyranoside

En opérant de façon analogue à l'exemple 3, mais en remplaçant le chloroformiate de propyle par le chloroformiate de 2-méthoxyéthyle, on obtient le produit attendu sous forme de poudre blanche légère (rendement = 80 %).
F = 118°C
[α]²³_{D} = - 69° (c = 0,65 ; CHCl₃)

### Exemple 7

### 4-Méthyl-2-oxo-2H-1-benzopyran-7-yl 2,3,4-tri-O-(2-propénylcarbonyl)-5-thio-β-D-xylopyranoside

En opérant de façon analogue à l'exemple 3, mais en remplaçant le chloroformiate de propyle par le chloroformiate d'allyle, on obtient le produit attendu sous forme d'un solide blanc pulvérulent (rendement = 8 %).
F = 120°C
[α]²²_{D} = - 57° (c = 0,46 ; CHCl₃)

### Exemple 8

### 4-Méthyl-2-oxo-2H-1-benzopyran-7-yl 2,3,4-tri-O-(éthènyloxycarbanyl)-5-thio-β-D-xylopyranoside

En opérant de façon analogue à l'exemple 3, mais en remplaçant le chloroformiate de propyle par le chloroformiate de vinyle, on obtient le produit attendu sous forme de poudre blanche (rendement = 57 %).
F = 120°C
[α]²³_{D} = -45° (c = 0,47 ; CHCl₃)

### PREPARATION I

### 2-[[(1,1-diméthyléthyl)diméthylsilyl]oxy]éthyl chloroformiate

On prépare une solution de 3,1 g (17,6 10⁻³ mole) de 2-[[(1,1-diméthyléthyl)diméthylsilyl]oxy]éthanol et 1,3 ml (19,3.10⁻³ mole) de pyridine dans 15 ml de dichlorométhane, et on ajoute goutte à goutte cette solution à un mélange de 10 ml d'une solution de phosgène à 20 % dans le toluène (soit 19,3.10⁻³ mole de phosgène) et 10 ml de dichlorométhane, refroidi à 0 °C. Après la fin de l'addition, le milieu réactionnel est maintenu sous agitation pendant 2 heures puis remis à température ambiante. On fait ensuite barboter dans le mélange un courant d'argon pendant 30 mn de façon à chasser le phosgène résiduel. La solution résiduelle est utilisée directement pour l'étape de synthèse suivante.

### PREPARATION II

### 4-Méthyl-2-oxo-2H-1-benzopyran-7-yl 2,3,4-tri-O-[[2-[[(1,1-diméthyléthyl)-diméthylsilyl]oxy]éthoxy]carbonyl]-5-thio-β-D-xylopyranoside

On prépare une solution de 500 mg (1,5.10⁻³ mole) de 4-méthyl-2-oxo-2*H*-1-benzopyran-7-yl 5-thio-β-D-xylopyranoside et 50 mg (0,41.10⁻³ mole) de 4-(diméthylamino)pyridine dans 25 ml de pyridine. Cette solution est ajoutée goutte à goutte à la solution de chloroformiate obtenue à la préparation I, maintenue sous agitation. Le mélange réactionnel est ensuite agité pendant 18 heures à température ambiante. On ajoute ensuite 50 ml de dichlorométhane et 100 ml d'eau. Les phases sont séparées et la phase aqueuse est extraite avec 50 ml de dichlorométhane. Les phases organiques rassemblées sont lavées à l'eau puis séchées sur sulfate de magnésium et concentrées sous pression réduite. Le résidu est purifié par chromatographie sur gel de silice en éluant à l'aide d'un mélange acétate d'éthyle/hexane (3/7 ; v/v). On obtient ainsi 1,13 g du produit attendu sous forme d'un solide pâteux jaune clair (rendement = 79 %).

### Exemple 9

### 4-Méthyl-2-oxo-2H-1-benzopyran-7-yl 2,3,4-tri-O-(2-hydroxyéthoxycarbonyl)-5-thio-β-D-xylopyranoside

On dissout 880 mg (0,95.10⁻³ mole) du composé obtenu selon la préparation II dans 40 ml d'acétonitrile et on ajoute 0,25 ml d'acide fluorhydrique à 40 %. Le mélange réactionnel est agité pendant 5 minutes à température ambiante puis concentré sous pression réduite. Le résidu est chromatographié sur gel de silice en éluant à l'aide d'un mélange dichlorométhane/méthanol (9/1 ; v/v). On obtient ainsi 486 mg du produit attendu sous forme d'une poudre blanche (rendement = 87 %).
F = 97-98 °C
[α]²⁵_{D} = - 76° (c = 0,35 ; CHCl₃)

L'activité pharmacologique de certains des composés selon l'invention a été mise en évidence au moyen d'un test reproduisant une thrombose veineuse et dont le protocole opératoire est le suivant :

L'expérimentation est réalisée sur des rats mâles Wistar, non à jeun, pesant 250 à 280 g et répartis en groupes de 10 animaux chacun. Les produits à tester sont administrés soit par voie orale (tubage) en solution ou en suspension dans le PEG 400, soit par injection intraveineuse, en solution dans le PEG 400. La concentration des composés est calculée de façon à faire absorber une quantité de solution de 2 ml/kg par voie orale et 1 ml/kg par injection intraveineuse. De façon générale, les composés sont administrés à la dose de 5 mg/kg par voie intraveineuse. Lorsque le test est effectué après administration par voie orale, la quantité administrée est calculée pour correspondre à environ 2.10⁻⁵ mole/kg. Une thrombose est induite à un temps T (2 h, 4 h ou 8 heures) après l'administration du produit et le thrombus formé est prélevé et pesé. Pour induire cette thrombose, on réalise une stase veineuse sous hypercoagulation, selon la technique décrite par WESSLER (J. Applied Physiol. 1959, p. 943-946) en utilisant en tant qu'agent hypercoagulant une solution de facteur X activé (Xa), fournie par la société Biogenic, et dosée à 7,5 nKat/kg. Les résultats obtenus, exprimés en pourcentage d'inhibition calculé par rapport au poids d'un thrombus obtenu en l'absence de principe actif dans le véhicule, sont consignés dans le tableau I ci-après. L'activité des composés testés a été contrôlée à différentes doses, après qu'ils aient été administrés soit par voie orale (p.o) soit par voie intraveineuse (i.v.). L'induction de la thrombose a été faite 4 heures ou 8 heures après l'administration du composé par voie orale et 2 heures après administration par voie intraveineuse. A titre de comparaison, on a également reporté dans le tableau I les résultats obtenus avec le 4-méthyl-2-oxo-2*H*-benzopyran-7-yl 5-thio-β-D-xylopyranoside, décrit dans EP-A-0421829 précité, et noté D dans ledit tableau I (ce composé est insoluble dans les solvants injectables, notamment l'eau et le PEG 400, et ne peut pas être administré par voie intraveineuse).

Les composés selon l'invention présentent, après administration par voie intraveineuse, une activité antithrombotique, sensiblement équivalente à celle obtenue par voie orale avec le produit de comparaison D, mais le délai d'action est plus rapide. Ces composés, qui conservent une bonne activité par voie orale peuvent être présentés soit sous forme injectable pour obtenir un effet rapide, soit sous une forme absorbable par voie orale dans le but d'éviter les désagréments ou les risques liés aux injections répétées.

Ils peuvent être formulés avec des excipients physiologiquement acceptables pour obtenir une forme injectable directement, une forme injectable à préparer extemporanément ou une forme solide pour voie orale telle que par exemple une gélule ou un comprimé, chaque unité contenant environ 25 à 500 mg d'au moins l'un des composés de formule I.

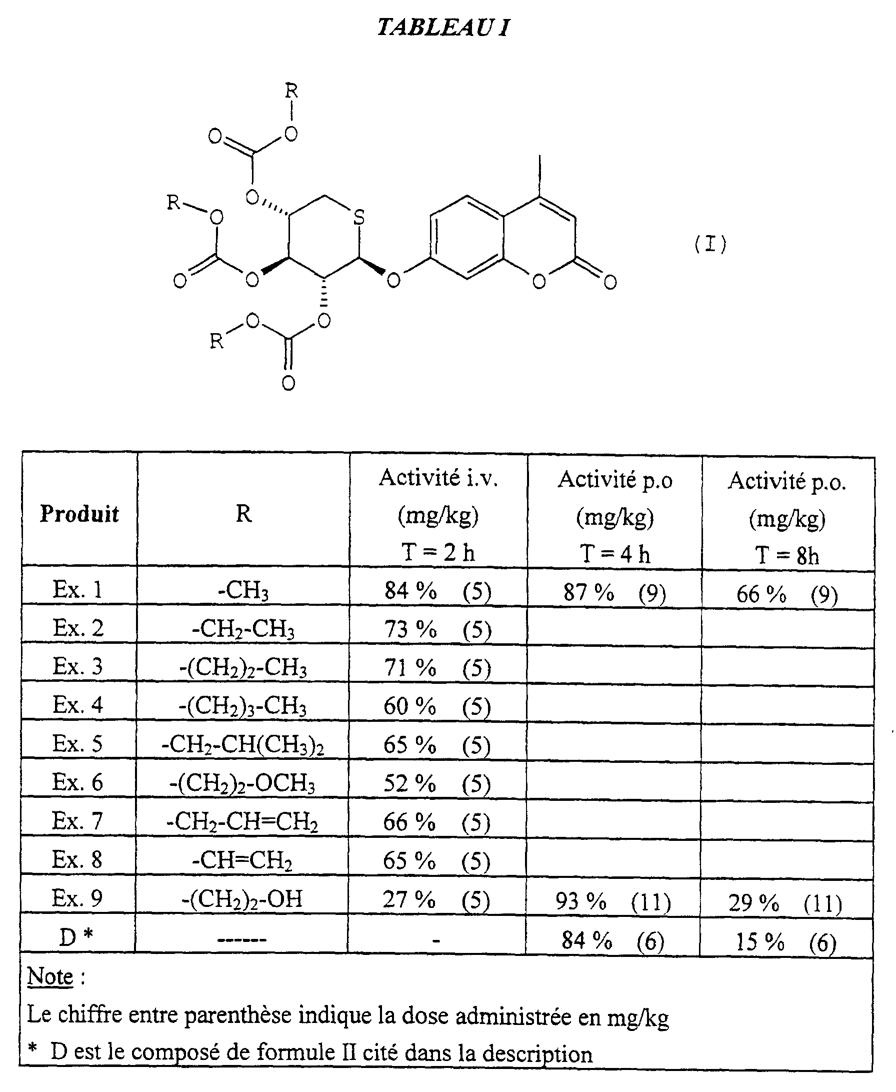

## Revendications

1. Composé de 4-méthyl-2-oxo-2*H*-1-benzopyran-7-yl 5-thio-β-D-xylopyranoside **caractérisé en ce qu'**il répond à la formule I : 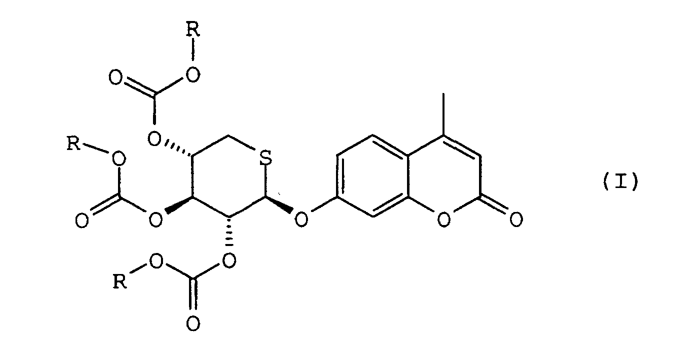 dans laquelle R représente un groupe alkyle en C₁-C₅ qui est linéaire, ramifié ou cyclisé, un groupe alcène en C₂-C₃ mono insaturé, un groupe hydroxyalkyle en C₂-C₃ ou un groupe alkoxyalkyle en C₃-C₆.

2. Composé selon la revendication 1, **caractérisé en ce que** R est méthyle, éthyle, propyle, 1-méthyléthyle, cyclopropyle, butyle, 1-méthylpropyle, 2-méthylpropyle, 1,1-diméthyléthyle ou pentyle.

3. Composé selon la revendication 1, **caractérisé en ce que** R est vinyle ou allyle.

4. Composé selon la revendication 1, **caractérisé en ce que** R est méthoxyéthyle, éthoxyéthyle, méthoxyéthoxyéthyle ou éthoxyéthoxyéthyle.

5. Composé selon la revendication 1, **caractérisé en ce qu'**il s'agit du 4-méthyl-2-oxo-2*H*-1-benzopyran-7-yl 2,3,4-tri-O-éthoxycarbonyl-5-thio-β-D-xylopyranoside.

6. Composé selon la revendication 1, **caractérisé en ce qu'**il s'agit du 4-méthyl-2-oxo-2*H*-1-benzopyran-7-yl 2,3,4-tri-O-méthoxycarbonyl-5-thio-β-D-xylopyranoside.

7. Procédé pour la préparation d'un composé de formule I suivant la revendication 1 dans lequel le groupe R représente un groupe alkyle en C₁-C₅ qui est linéaire, ramifié ou cyclisé, un groupe alcène en C₂-C₃ mono insaturé ou un groupe alkoxyalkyle en C₃-C₆, ledit procédé étant **caractérisé en ce qu'**il comprend la réaction de carbonatation du 4-méthyl-2-oxo-2*H*-1-benzopyran-7-yl 5-thio-β-D-xylopyranoside de formule II : 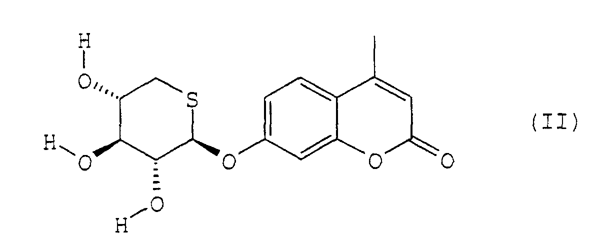 avec un réactif choisi parmi l'ensemble constitué par un halogénoformiate de formule III : 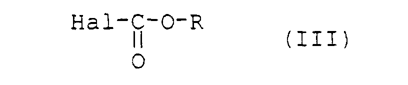 et un pyrocarbonate de formule IV : 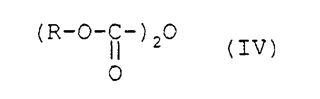 où R représente un groupe alkyle en C₁-C₅ qui est linéaire, ramifié ou cyclisé, un groupe alcène en C₂-C₃ mono insaturé ou un groupe alkoxyalkyle en C₃-C₆ et Hal est un atome d'halogène (notamment F, Cl ou Br).

8. Procédé pour la préparation d'un composé de formule I dans lequel R représente un groupe hydroxyalkyle en C₂-C₃, ledit procédé étant **caractérisé en ce qu'**il comprend la réaction de carbonatation du 4-méthyl-2-oxo-2*H*-1-benzopyran-7-yl 5-thio-β-D-xylopyranoside de formule II : 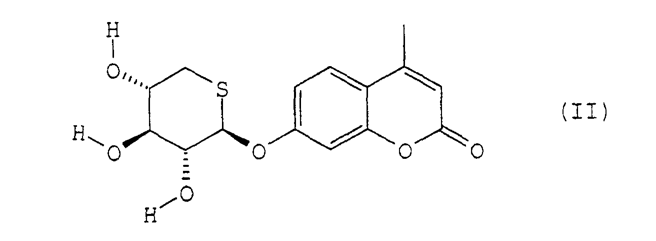 avec un halogénoformiate de formule IIIbis : 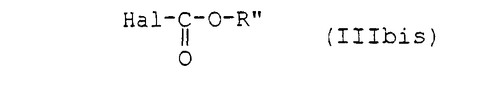 où R" est un groupe hydroxyalkyle en C₂-C₃ dans lequel la fonctionnalité OH est protégée, et Hal est un atome d'halogène comme défini ci-dessus, puis l'on soumet le composé résultant à une réaction de déprotection de la fonctionnalité OH du groupe hydroxyalkyle, par action d'une solution d'acide fluorhydrique afin d'obtenir le composé de formule I dans laquelle R représente le groupe hydroxyalkyle en C₂-C₃, dans un solvant anhydre, en présence d'une base aprotique, à une température de 10°C à 80°C et pendant une durée de 1 à 10 heures.

9. Composition pharmaceutique **caractérisée en ce qu'**elle comprend, en association avec un excipient physiologiquement acceptable, une quantité thérapeutiquement efficace d'au moins un composé de formule I suivant la revendication 1.

10. Utilisation d'un composé de formule I suivant la revendication 1, **caractérisée en ce que** l'on fait appel à un dit composé de formule I pour l'obtention d'un médicament antithrombotique destiné à une utilisation thérapeutique vis-à-vis des troubles de la circulation veineuse.

## Claims

1. 4-Methyl-2-oxo-2*H*-1-benzopyran-7-yl 5-thio-β-D-xylopyranoside compound, **characterized in that** it has formula I: 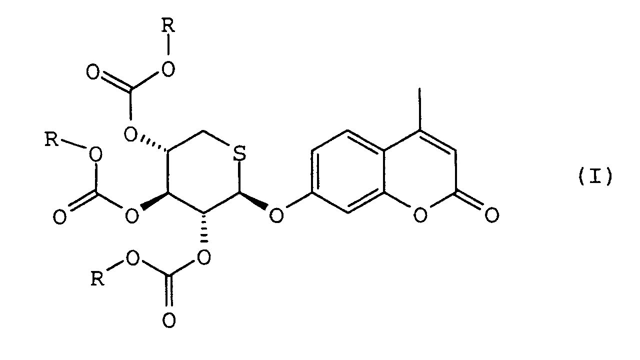 in which R is a linear, branched or cyclic C₁-C₅-alkyl group, a mono-unsaturated C₂-C₃-alkene group, a C₂-C₃-hydroxyalkyl group or a C₃-C₆-alkoxyalkyl group.

2. Compound according to claim 1, **characterized in that** R is methyl, ethyl, propyl, 1-methylethyl, cyclopropyl, butyl, 1-methylpropyl, 2-methylpropyl, 1,1-dimethylethyl or pentyl.

3. Compound according to claim 1, **characterized in that** R is vinyl or allyl.

4. Compound according to claim 1, **characterized in that** R is methoxyethyl, ethoxyethyl, methoxyethoxyethyl or ethoxyethoxyethyl.

5. Compound according to claim 1, **characterized in that** it is 4-methyl-2-oxo-2*H*-1-benzopyran-7-yl 2,3,4-tri-O-ethoxycarbonyl-5-thio-β-D-xylopyranoside.

6. Compound according to claim 1, **characterized in that** it is 4-methyl-2-oxo-2*H*-1-benzopyran-7-yl 2,3,4-tri-O-methoxycarbonyl-5-thio-β-D-xylopyranoside.

7. Process for the preparation of a compound of formula I according to claim 1 in which the group R is a linear, branched or cyclic C₁-C₅-alkyl group, a mono-unsaturated C₂-C₃-alkene group or a C₃-C₆-alkoxyalkyl group, said process being **characterized in that** it comprises the carbonation reaction of 4-methyl-2-oxo-2*H*-1-benzopyran-7-yl 5-thio-β-D-xylopyranoside of formula II: 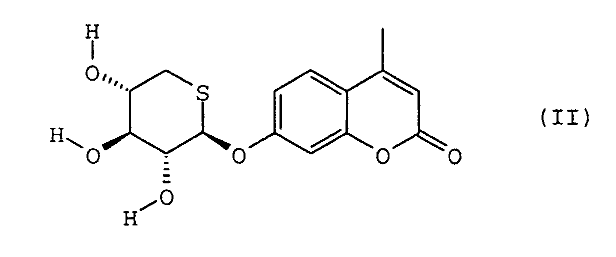 with a reagent selected from the group consisting of a halogenoformate of formula III: 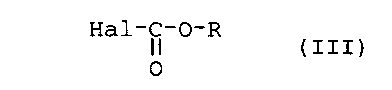 and a pyrocarbonate of formula IV: 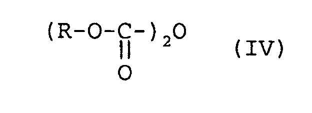 in which R is a linear, branched or cyclic C₁-C₅-alkyl group, a mono-unsaturated C₂-C₃-alkene group or a C₃-C₆-alkoxyalkyl group and Hal is a halogen atom (especially F, Cl or Br).

8. Process for the preparation of a compound of formula I in which R is a C₂-C₃-hydroxyalkyl group, said process being **characterized in that** it comprises the carbonation reaction of 4-methyl-2-oxo-2*H*-1-benzopyran-7-yl 5-thio-β-D-xylopyranoside of formula II: 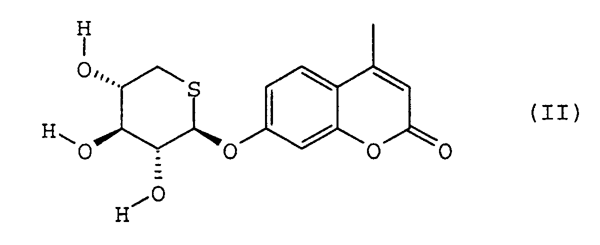 with a halogenoformate of formula IIIbis: 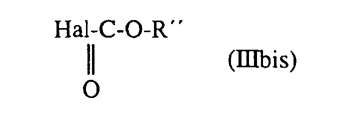 in which R" is a C₂-C₃-hydroxyalkyl group with its OH group protected and Hal is a halogen atom as defined above, and the resulting compound is then subjected to a deprotection reaction of the OH group of the hydroxyalkyl group by reaction with hydrofluoric acid solution to give the compound of formula I in which R is a C₂-C₃-hydroxyalkyl group, in an anhydrous solvent, in the presence of an aprotic base, at a temperature of 10°C to 80°C for a period of 1 to 10 hours.

9. Pharmaceutical composition, **characterized in that** it comprises a therapeutically effective amount of at least one compound of formula I according to claim 1 in association with a physiologically acceptable excipient.

10. Use of a compound of formula I according to claim 1, **characterized in that** said compound of formula I is used for obtaining an antithrombotic drug intended for therapeutic use to combat disorders of the venous circulation.

## Patentansprüche

1. 4-Methyl-2-oxo-2*H*-1-benzopyran-7-yl-5-thio-β-D-xylopyranosid-Verbindung **gekennzeichnet durch** die Formel I: 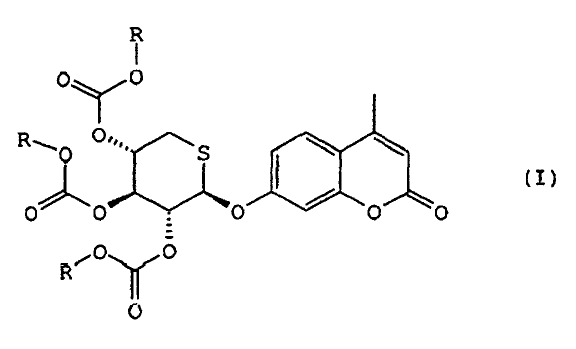 wobei R einen geradkettigen, verzweigten oder cyclischen C₁-C₅-Alkylrest, einen einfach ungesättigten C₂-C₃-Alkenrest, einen C₂-C₃-Hydroxyalkylrest oder einen C₃-C₆-Alkoxyalkylrest bedeutet.

2. Verbindung gemäß Anspruch 1, **dadurch gekennzeichnet, dass** R eine Methyl-, Ethyl-, Propyl-, 1-Methylethyl-, Cyclopropyl-, Butyl-, 1-Metylpropyl-, 2-Methylpropyl-, 1,1-Dimethylethyl- oder Pentylgruppe bedeutet.

3. Verbindung gemäß Anspruch 1, **dadurch gekennzeichnet, dass** R eine Vinyl- oder Allylgruppe bedeutet.

4. Verbindung gemäß Anspruch 1, **dadurch gekennzeichnet, dass** R eine Methoxyethyl-, Ethoxyethyl-, Methoxyethoxyethyl- oder Ethoxyethoxyethylgruppe bedeutet.

5. Verbindung gemäß Anspruch 1, **dadurch gekennzeichnet, dass** es sich dabei um 4-Methyl-2-oxo-2*H*-1-benzopyran-7-yl-2,3,4-tri-O-ethoxycarbonyl-5-thio-β-Dxylopyranosid handelt.

6. Verbindung gemäß Anspruch 1, **dadurch gekennzeichnet, dass** es sich dabei um 4-Methyl-2-oxo-2*H*-1-benzopyran-7-yl-2,3,4-tri-O-methoxycarbonyl-5-thio-β-Dxylopyranosid handelt.

7. Verfahren zur Herstellung einer Verbindung der Formel I gemäß Anspruch 1, wobei der Rest R einen geradkettigen, verzweigten oder cyclischen C₁-C₅-Alkylrest, einen einfach ungesättigten C₂-C₃-Alkenrest oder einen C₃-C₆-Alkoxyalkylrest bedeutet, wobei das Verfahren **dadurch gekennzeichnet ist, dass** es die Carbonatisierungsreaktion des 4-Methyl-2-oxo-2*H*-1-benzopyran-7-yl-5-thio-β-D-xylopyranosids der Formel II: 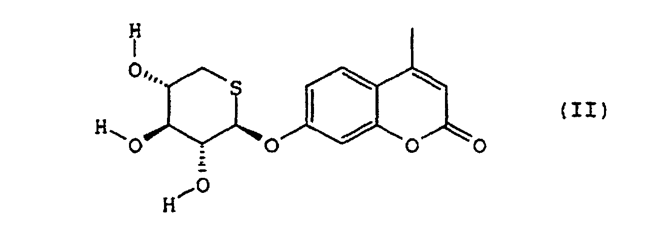 mit einem Reagens, das aus einem Halogenformiat der Formel III: 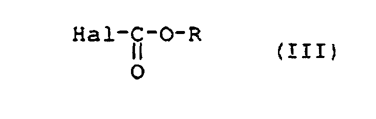 und einem Pyrocarbonat der Formel IV: 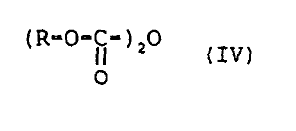 ausgewählt ist, umfasst, wobei R einen geradkettigen, verzweigten oder cyclischen C₁-C₅-Alkylrest, einen einfach ungesättigten C₂-C₃-Alkenrest oder einen C₃-C₆-Alkoxyalkylrest und Hal ein Halogenatom (insbesondere F, Cl oder Br) bedeutet.

8. Verfahren zur Herstellung einer Verbindung der Formel I, wobei R einen C₂-C₃-Hydroxyalkylrest bedeutet, wobei das Verfahren **dadurch gekennzeichnet ist, dass** es die Carbonatisierungsreaktion des 4-Methyl-2-oxo-2*H*-1-benzopyran-7-yl-5-thio-β-D-xylopyranosids der Formel II: 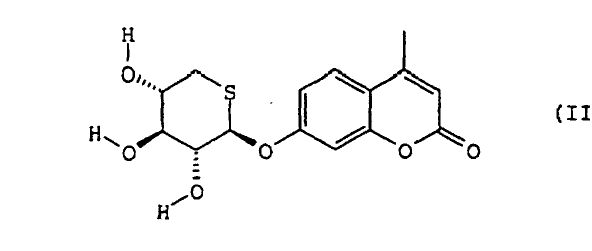 mit einem Halogenformiat der Formel IIIbis: 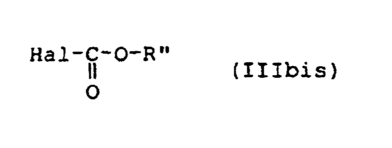 umfasst, wobei R" einen C₂-C₃-Hydroxyalkylrest mit geschützter OH-Funktion und Hal ein wie vorstehend angegeben definiertes Halogenatom bedeutet, und die erhaltene Verbindung wird anschließend einer Entschützungsreaktion der OH-Funktion des Hydroxyalkylrests durch Einwirkung einer Lösung von Fluorwasserstoffsäure unterzogen, um die Verbindung der Formel I zu erhalten, in der R den C₂-C₃-Hydroxyalkylrest bedeutet, in einem wasserfreien Lösungsmittel in Gegenwart einer aprotischen Base bei einer Temperatur von 10°C bis 80°C und für eine Dauer von 1 bis 10 Stunden.

9. Arzneimittel, **dadurch gekennzeichnet, dass** es eine therapeutisch wirksame Menge mindestens einer Verbindung der Formel I gemäß Anspruch 1 in Verbindung mit einem physiologisch verträglichen Exzipienten umfasst.

10. Verwendung einer Verbindung der Formel I gemäß Anspruch 1, **dadurch gekennzeichnet, dass** auf die Verbindung der Formel I zurückgegriffen wird, um ein antithrombotisches Medikament zu erhalten, das zu einer therapeutischen Verwendung bei Problemen der venösen Zirkulation vorgesehen ist.
